# EUROPEAN PATENT SPECIFICATION

(11) **EP 1 732 894 B1**
(45) Date of publication and mention of the grant of the patent: **27.06.2007**
(21) Application number: 05728224.6
(22) Date of filing: 26.03.2005
(51) Int. Cl.: C07D 213/53, C07B 43/00

(54) **PROCESS FOR PREPARING DIIMINE COMPOUNDS**
VERFAHREN ZUR HERSTELLUNG VON DIIMINVERBINDUNGEN
PROCEDÉ DE PREPARATION DE COMPOSÉS DE DIIMINE

(30) Priority: 08.04.2004 DE 102004018043; 07.05.2004 US 569142 P
(43) Date of publication of application: 20.12.2006
(73) Proprietor: Basell Polyolefine GmbH, 50389 Wesseling (DE)
(72) Inventor: KÖLLING, Lars, 68165 Mannheim (DE)
(86) International application number: PCT/EP2005/003206
(87) International publication number: WO 2005/097712

(56) References cited:
- WO-A-01/07491
- WO-A-98/27124
- C QIAN ET AL.: "Silica-Alun^minia Catalyst Support, an Efficient Catalyst for Synthesis of Halogen Substituted 2,6-Bis(imino)pyridines" SYNLETT, no. 10, 5 August 2003 (2003-08-05), XP002370866 cited in the application
- PATENT ABSTRACTS OF JAPAN vol. 1999, no. 03, 31 March 1999 (1999-03-31) & JP 10 324689 A (SHIN ETSU CHEM CO LTD), 8 December 1998 (1998-12-08)

## Description

The present invention relates to a process for preparing diimine compounds.

The use of metallocene catalysts in the polymerization of unsaturated compounds has had a great influence on the preparation of polyolefins, since it opens up a route to new types of polyolefinic materials or to materials having improved properties. There is therefore great interest in the development of new families of catalysts for the polymerization of unsaturated compounds in order to obtain even better control over the properties of polyolefins or to obtain further novel products.

In particular, the use of transition metal catalysts comprising late transition metals is of interest because they are able to tolerate heteroatom functions. Transition metal catalysts comprising late transition metals which are suitable for the polymerization of unsaturated compounds are known from the prior art. Here, 1,2-diiminenickel and 2,6-bis(imino)pyridyliron complexes have been found to be particularly useful.

The ligand systems such as 1,2-diimine or 2,6-bis(imino)pyridyl compounds are usually prepared via condensation of the corresponding diketo compounds with primary amines. Since in this reaction two keto functions have to be converted into the corresponding imines, the yields of diimine compound are often low. Particularly when primary amines having electron-withdrawing or bulky groups are used, the yield of diimine product drops: It is often the case here that only one of the two keto functions is converted into the imine. Furthermore, the reaction time usually has to be increased significantly in order to obtain at least a small yield of the diimine.

WO 98/27124 discloses the synthesis of 2,6-bis(imino)pyridyl compounds from the corresponding diketo compounds and anilines in methanol with addition of catalytic amounts of formic acid.
When anilines bearing a halogen in the 2 position are used, the yield is only very low.

WO 01/07491 describes the synthesis of 2,6-bis(imino)pyridyl compounds from the corresponding keto compounds and ortho-halogen-substituted anilines in benzene in the presence of catalytic amounts of p-toluenesulfonic acid using a water separator. Here too, the yield of 2,6-bis(imino)pyridyl compound is below 50%.

In Synlett. 2003, (10), pp.1419-1422, C. Qian et al. compares a number of synthetic routes for preparing halogen-substituted 2,6-bis(imino)pyridyl compounds. The best results are achieved by means of a combination of Al-Si-catalyst and 4 Å molecular sieves.

It is an object of the present invention to provide an improved process for synthesizing diimine compounds, by means of which even primary amines having electron-withdrawing or bulky substituents can be converted into the corresponding diimine compounds in high yields.

We have accordingly found a process for preparing diimine compounds wherein a dicarbonyl compound is reacted with primary amines in the presence of phosphorus pentoxide.

In the process of the invention, a diimine compound is any compound which contains at least two imine groups -C=N- which is not part of a heteroaromatic ring.

Preference is given to diimine compounds of the formula I 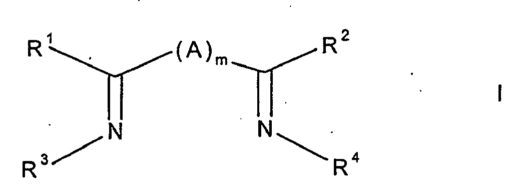 where the variables have the following meanings:
- R¹-R⁴: are each, independently of one another, hydrogen, C₁-C₂₂-alkyl, C₂-C₂₂-alkenyl, . C₆-C₂₂-aryl, alkylaryl having from 1 to 10 carbon atoms in the alkyl part and 6-20 carbon atoms in the aryl part, NR⁵₂, or a five-, six- or seven-membered heterocycle containing at least one atom from the group consisting of N, P, O and S, where the organic radicals R¹-R⁴ may also be substituted by halogens, NR⁵₂, OR⁵ or SiR⁶₃ and/or two radicals R¹-R⁴ may also be joined with one another or with A to form a ring,
- the radicals R⁵: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR⁶₃, where the organic radicals R⁵ may also be substituted by halogens and two radicals R⁵ may also be joined to form a five- or six-membered ring, and
- the radicals R⁶: are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R⁶ may also be joined to form a five- or six-membered ring,
- m: is 0 or 1, '
- A: is 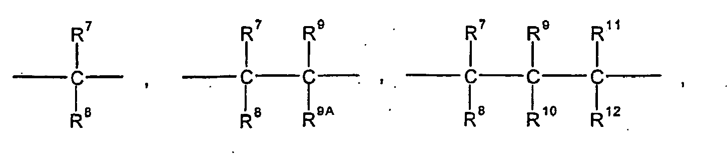 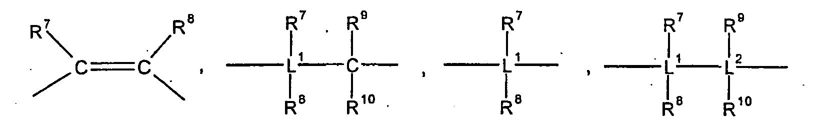 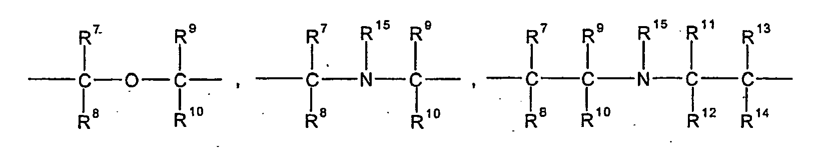 or a heteroaromatic ring system, where
- L¹-L²: are each, independently of one another, silicon or germanium,
- R⁷-R¹⁵: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR¹⁶₃, where the organic radicals R⁷-R¹⁵ may also be substituted by halogens and two radicals R⁷-R¹⁵ may also be joined to form a five- or six-membered ring, and
- the radicals R¹⁶: are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R¹⁶ may also be joined to form a five- or six-membered ring.

In the process of the invention, a dicarbonyl compound is any compound which contains at least two keto groups, -C=O, which are not part of a heteroaromatic ring, with aldehyde groups also being included.

Preference is given to dicarbonyl compounds of the formula II 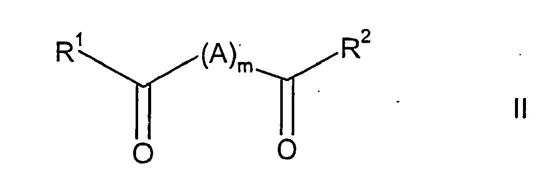 where the variables have the following meanings:
- R¹-R²: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, NR⁵₂, or a five-, six- or seven-membered heterocycle containing at least one atom from the group consisting of N, P, O and S, where the organic radicals R¹-R⁴ may also be substituted by halogens, NR⁵₂, OR⁵ or SiR⁶₃ and/or two radicals R¹-R² may also be joined with one another or with A to form a ring,
- the radicals R⁵: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR⁶_{3'} where the organic radicals R⁵ may also be substituted by halogens and two radicals R⁵ may also be joined to form a five- or six-membered ring, and
- the radicals R⁶: are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R⁶ may also be joined to form a five- or six-membered ring,
- m: is 0 or 1,
- A: is 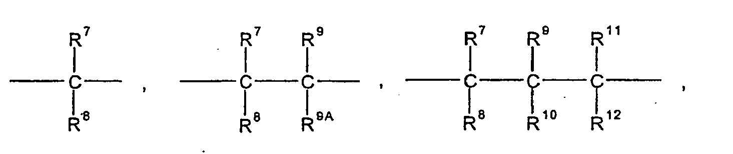 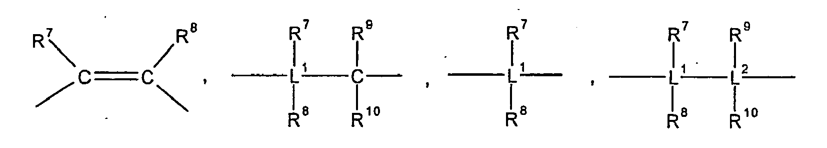 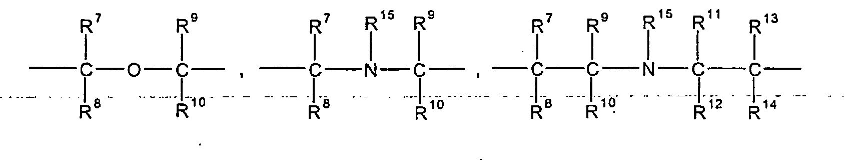 or a heteroaromatic ring system, where
- L¹-L²: are each, independently of one another, silicon or germanium,
- R⁷-R¹⁵: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR¹⁶₃, where the organic radicals R⁷-R¹⁵ may also be substituted by halogens and two radicals R⁷-R¹⁵ may also be joined to form a five- or six-membered ring, and
- the radicals R¹⁶: are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R¹⁶ may also be joined to form a five- or six-membered ring.

In the process of the invention, a primary amine is any compound which bears an NH₂ group, i.e. including hydrazines.

The process of the invention is preferably employed for preparing diimine compounds of the formula I by reacting the dicarbonyl compound of the formula II with primary amines in the presence of phosphorus pentoxide. Primary amines used here are NH₂R³, and NH₂R⁴, where the meanings of R³ and R⁴ and their preferred embodiments correspond to those given for the diimine compound of the formula I. Preference is given to NH₂R³ and NH₂R⁴ being identical, so that R³ and R⁴ in the diimine compound I are also identical. Examples of primary amines are methylamine, ethylamine, n-propylamine, isopropylamine, n-butylamine, tert-butylamine, sec-butylamine, isobutylamine, tert-amylamine, n-pentylamine, n-hexylamine, n-octylamine, cyclohexylamine, aniline, 2-methylaniline, 2-chloroaniline, 2-bromoaniline, 2,6-dichloroaniline, 2,4-dichloro-6-methylaniline and 2,6-dibromoaniline. R³ and R⁴ preferably contain a halogen-containing substituent.

The substituents R¹-R² of the diimine compound of the formula I and of the dicarbonyl compound of the formula II and R³-R⁴ of the diimine compound of the formula I can be varied within wide ranges. Possible carboorganic substituents R¹-R⁴ are, for example, the following: C₁-C₂₂-alkyl which may be linear or branched, e.g. methyl, ethyl, n-propyl, isopropyl, n-butyl, isobutyl, tert-butyl, n-pentyl, n-hexyl, n-heptyl, n-octyl, n-nonyl, n-decyl or n-dodecyl, 5- to 7-membered cycloalkyl which may in turn bear a C₁-C₁₀-alkyl group and/or C₆-C₁₀-aryl group as substituent, e.g. cyclopropyl, cyclobutyl, cyclopentyl, cyclohexyl, cycloheptyl, cyclooctyl, cyclononyl or cyclododecyl, C₂-C₂₂-alkenyl which may be linear, cyclic or branched and in which the double bond may be internal or terminal, e.g. vinyl, 1-allyl, 2-allyl, 3-allyl, butenyl, pentenyl, hexenyl, cyclopentenyl, cyclohexenyl, cyclooctenyl or cyclooctadienyl, C₆-C₂₂-aryl which may be substituted by further alkyl groups, e.g. phenyl, naphthyl, biphenyl, anthranyl, o-, m-, p-methylphenyl, 2,3-, 2,4-, 2,5- or 2,6-dimethylphenyl, 2,3,4-, 2,3,5-, 2,3,6-, 2,4,5-, 2,4,6- or 3,4,5-trimethylphenyl, or arylalkyl, which may bear further alkyl groups as substituents, e.g. benzyl, o-, m-, p-methylbenzyl, 1- or 2-ethylphenyl, where, if appropriate, two radicals R¹-R² may also be joined to one another or to A to form a 5-, 6-, 7- or 8-membered ring which may also be a heterocycle containing at least one atom from the group consisting of N, P, O and S. Furthermore, R¹-R⁴ can be amino NR⁵₂, for - example dimethylamino, N-pyrrolidinyl, diphenylamino or picolinyl. In this amino group, it is preferred that none of the substituents R⁵ is hydrogen. The organic radicals R¹-R⁴ may also be substituted by halogens such as fluorine, chlorine or bromine, by amino NR⁵₂, for example dimethylamino, N-pyrrolidinyl or picolinyl, by alkoxy or aryloxy OR⁵, e.g. methoxy, ethoxy or isopropoxy, or organosilicon substituents SiR⁶₃, e.g. trimethylsilyl, triethylsilyl, butyldimethylsilyl, tributylsilyl, tri-tert-butylsilyl, triallylsilyl, triphenylsilyl or dimethylphenylsilyl. Possible substituents R⁵ are the same carboorganic radicals as described in more detail above for R¹-R⁴, where, if appropriate, two R⁵ radicals may be joined to form a 5- or 6-membered ring and/or be substituted by halogen. A possible radical R⁶ in organosilicon substituents SiR⁶₃ are the same carboorganic radicals as described in more detail above for R¹-R⁴, where, if appropriate, two R⁶ radicals may also be joined to form a 5- or 6-membered ring. Preference is given to R³ and R⁴ being identical.

A is a bridge between the two keto or imino groups, preferably comprising carbon- and/or silicon-and/or n-containing bridge members. The activity of the catalyst can be influenced by a change in the length of the linkage between the imino groups.

Possible carboorganic substituents R⁷-R¹⁵ in the linkage A are, for example, the following: hydrogen, C₁-C₂₂-alkyl which may be linear or branched, e.g. methyl, ethyl, n-propyl, isopropyl, n-butyl, isobutyl, tert-butyl, n-pentyl, n-hexyl, n-heptyl, n-octyl, n-nonyl, n-decyl or n-dodecyl, 5- to 7-membered cycloalkyl which may in turn bear a C₆-C₁₀-aryl group as substituent, e.g. cyclopropyl, cyclobutyl, cyclopentyl, cyclohexyl, cycloheptyl, cyclooctyl, cyclononyl or cyclododecyl, C₂-C₂₂-alkenyl which may be linear, cyclic or branched and in which the double bond may be internal or terminal, e.g. vinyl, 1-allyl, 2-allyl, 3-allyl, butenyl, pentenyl, hexenyl, cyclopentenyl, cyclohexenyl, cyclooctenyl or cyclooctadienyl, C₆-C₂₀-aryl which may be further alkyl groups substituted by, e.g. phenyl, naphthyl, biphenyl, anthranyl, o-, m-, p-methylphenyl, 2,3-, 2,4-, 2,5-or 2,6-dimethylphen-1-yl, 2,3,4-, 2,3,5-, 2,3,6-, 2,4,5-, 2,4,6- or 3,4,5-trimethylphen-1-yl, or arylalkyl, which may bear further alkyl groups as substituents, e.g. benzyl, o-, m-, p-methylbenzyl, 1- or 2-ethylphenyl, where, if appropriate, two radicals R⁷ to R¹⁵ may also be joined to form a 5- or 6-membered ring, cyclohexane, and the organic radicals R⁷-R¹⁵ may also be substituted by halogens such as fluorine, chlorine or bromine, for example pentafluorophenyl or bis-3,5-trifluoro-methylphen-1-yl. Preferred radicals R⁷-R¹⁵ are hydrogen, methyl, ethyl, n-propyl, isopropyl, n-butyl, isobutyl, tert-butyl, n-pentyl, n-hexyl, n-heptyl, n-octyl, benzyl, phenyl, ortho dialkyl- or dichloro-substituted phenyls, trialkyl- or trichloro-substituted phenyls, naphthyl, biphenyl and anthranyl.

Possible radicals R¹⁶ in organosilicon substituents SiR¹⁶₃ are the same radicals as described in more detail above for R⁷-R¹⁵, where, if appropriate, two R¹⁶ may also be joined to form a 5- or 6-membered ring, e.g. trimethylsilyl, triethylsilyl, butyldimethylsilyl, tributylsilyl, tri-tert-butylsilyl, triallylsilyl, triphenylsilyl or dimethylphenylsilyl.

A can also be a heteroaromatic ring system containing at least one atom from the group consisting of nitrogen, phosphorus, oxygen and sulfur, particularly preferably nitrogen and sulfur. Preference is given to heteroaromatics having a ring size of 5 or 6 ring atoms. Examples of 5-membered heterocycles which may contain from one to three nitrogen atoms and/or a sulfur or oxygen atom in addition to carbon atoms are 1,2-dihydrofuran, furan, thiophene, pyrrole, isoxazole, 3-isothiazole, pyrazole, oxazole, thiazole, imidazole, 1,2,4-oxadiazole, 1,2,5-oxadiazole, 1,3,4-oxadiazole, 1,2,3-triazole or 1,2,4-triazole. Examples of 6-membered heteroaryl groups which may contain from one to four nitrogen atoms and/or a phosphorus atom are pyridine, phospha-benzene, pyridazine, pyrimidine, pyrazine, 1,3,5-triazine, 1,2,4-triazine and 1,2,3-triazine. The 5-membered and 6-membered heterocycles can also be substituted by C₁-C₁₀-alkyl, C₆-C₁₀-aryl, alkylaryl having from 1 to 10 carbon atoms in the alkyl part and 6-10 carbon atoms in the aryl part, trialkylsilyl or halogens such as fluorine, chlorine or bromine, dialkylamide, alkylarylamide, diarylamide, alkoxy or aryloxy or be fused with one or more aromatics or heteroaromatics. Examples of benzo-fused 5-membered heteroaryl groups are indole, indazole, benzofuran, benzothiophene, benzothiazole, benzoxazole and benzimidazole. Examples of benzo-fused 6-membered heteroaryl groups are chromane, benzopyran, quinoline, isoquinoline, cinnoline, phthalazine, quinazoline, quinoxaline,1,10-phenanthroline and quinolizine. Nomenclature and numbering of the heterocycles has been taken from Lettau, Chemie der Heterocyclen, 1st edition, VEB, Weinheim 1979. The two keto or imino groups are preferably located on the heteroaromatic ring system in the ortho positions relative to the same heteroatom, preferably a nitrogen atom.

A is preferably a -CR⁷R⁸- group, substituted or unsubstituted 1,2-phenylene or - CR⁷R⁸CR⁹R¹⁰NR¹⁵CR¹¹R¹²CR¹³R¹⁴- . The above-described preferred embodiments of the substituents R⁷ to R¹⁵ are likewise preferred embodiments here. Preference is given to -CR⁷R⁸- being a -CHR⁸-, -CH₂- or -C(CH₃)₂- group.

In a further, preferred embodiment, A has the formula III 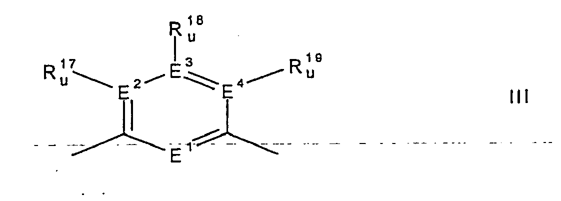 where the variables have the following meanings:
- E¹: is nitrogen or phosphorus, in particular nitrogen,
- E²-E⁴: are each, independently of one another, carbon, nitrogen or phosphorus, in particular carbon,
- R¹⁷-R¹⁹: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, halogen, NR²⁰₂, OR²⁰, SiR²¹₃, where the organic radicals R¹⁷-R¹⁹ may also be substituted by halogens and/or two vicinal radicals R¹⁷-R¹⁹ may also be joined to form a five-, six- or seven-membered ring, and/or two radicals R¹⁷-R¹⁹ are joined to form a five-, six- or seven-membered heterocycle containing at least one atom from the group consisting of N, P, O and S,
- the radicals R²⁰: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR²¹₃, where the organic radicals R²⁰ may also be substituted by halogens and two radicals R²⁰ may also be joined to form a five- or six-membered ring, and
- the radicals R²¹: are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R²¹ may also be joined to form a five- or six-membered ring,
- u: is 0 when E²-E⁴ is nitrogen or phosphorus and is 1 when E²-E⁴ is carbon.

The process of the invention has been found to be particularly useful for diimines of the formula I in which m is 0, known as 1,2-diimine systems, and those in which m is 1 and A has the formula III. The dicarbonyl compounds of the formula II from which these are prepared have the same preferred embodiments of the variables m and A.
The process of the invention is especially useful for preparing diimines of the formula IV 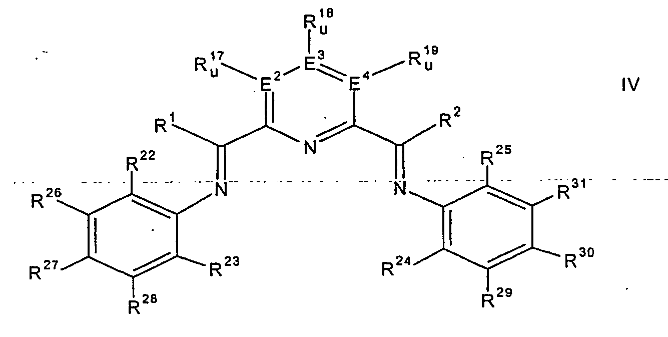 by reacting a dicarbonyl compound of the formula V 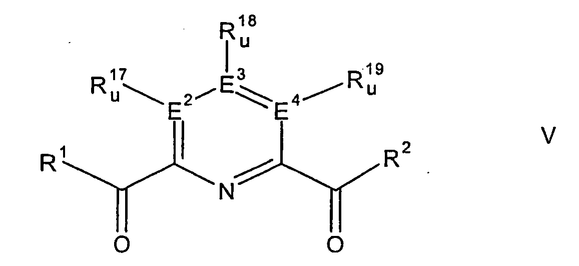 with primary amines in the presence of phosphorus pentoxide,
where the variables have the following meanings:
- E²-E⁴: are each, independently of one another, carbon or nitrogen, in particular carbon,
- R¹-R²: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, where the organic radicals R¹-R² may also be substituted by halogens, NR⁵₂, OR⁵ or SiR⁶₃ and/or the radicals R¹-R² may also be joined to R¹⁷-R¹⁹ to form a ring,
- R¹⁷-R¹⁹: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, halogen, NR²⁰₂, OR²⁰, SiR²¹₃, where the organic radicals R¹⁷-R¹⁹ may also be substituted by halogens and/or two vicinal radicals R¹⁷-R¹⁹ may also be joined to form a five-, six- or seven-membered ring, and/or two radicals R¹⁷-R¹⁹ are joined to form a five-, six- or seven-membered heterocycle containing at least one atom from the group consisting of N, P, O and S,
- the radicals R²⁰: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR²¹₃, where the organic radicals R²⁰ may also be substituted by halogens and two radicals R²⁰ may also be joined to form a five- or six-membered ring, and
- the radicals R²¹: are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R²¹ may also be joined to form a five- or six-membered ring,
- u: is 0 when E²-E⁴ is nitrogen or phosphorus and is 1 when E²-E⁴ is carbon,
- R²²-R³¹: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, halogen, NR³²₂, OR³², SiR³³₃, where the organic radicals R²²-R³¹ may also be substituted by halogens and/or two vicinal radicals R²²-R³¹ may also be joined to form a five-, six- or seven-membered ring, and/or two vicinal radicals R²²-R³¹ are joined to form a five-, six- or seven-membered heterocycle containing at least one atom from the group consisting of N, P, O and S,
- the radicals R³²: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, SiR³³₃, where the organic radicals R³² may also be substituted by halogens or nitrogen- and oxygen-containing groups and two radicals R³² may also be joined to form a five- or six-membered ring, and
- the radicals R³³: are each, independently of one another, hydrogen, C₁-C₂₀-alkyl, C₂-C₂₀-alkenyl, C₆-C₂₀-aryl, alkylaryl having from 1 to 10 carbon atoms in the alkyl part and 6-20 carbon atoms in the aryl part, where the organic radicals R³³ may also be substituted by halogens or nitrogen- and oxygen-containing groups and two radicals R³³ may also be joined to form a five- or six-membered ring.

The three atoms E² to E⁴ in a molecule can be identical or different. E² to E⁴ are each nitrogen or carbon, in particular carbon.

The substituents R¹-R² can be varied within wide ranges. Possible carboorganic substituents R^{1c}-R^{3c} and R^{8c}-R^{17c} are, for example, the following: C₁-C₂₂-alkyl which may be linear or branched, e.g. methyl, ethyl, n-propyl, isopropyl, n-butyl, isobutyl, tert-butyl, n-pentyl, n-hexyl, n-heptyl, n-octyl, n-nonyl, n-decyl or n-dodecyl, 5- to 7-membered cycloalkyl which may in turn bear a C₁-C₁₀-alkyl group and/or C₆-C₁₀-aryl group as substituent, e.g. cyclopropyl, cyclobutyl, cyclopentyl, cyclohexyl, cycloheptyl, cyclooctyl, cyclononyl or cyclododecyl, C₂-C₂₂-alkenyl, which may be linear, cyclic or branched and in which the double bond may be internal or terminal, e.g. vinyl, 1-allyl, 2-allyl, 3-allyl, butenyl, pentenyl, hexenyl, cyclopentenyl, cyclohexenyl, cyclooctenyl or cyclooctadienyl, C₆-C₂₂-aryl which may bear further alkyl groups as substituents, e.g. phenyl, naphthyl, biphenyl, anthranyl, o-, m-, p-methylphenyl, 2,3-, 2,4, 2,5- or 2,6-dimethylphenyl, 2,3,4-2,3,5-, 2,3,6-, 2,4,5-, 2,4,6- or 3,4,5-trimethylphenyl, or arylalkyl, which may bear further alkyl groups as substituents, e.g. benzyl, o-, m-, p-methylbenzyl, 1- or 2-ethylphenyl, where, if appropriate, a radical R¹ to R² may be joined to R¹⁷-R¹⁹ to form a 5-, 6- or 7-membered ring and/or the radicals R¹-R² may be joined to the radicals R¹⁷-R¹⁹ to form a five-, six- or seven-membered heterocycle containing at least one atom from the group consisting of N, P, O and S and/or the organic radicals R¹-R² may also be substituted by halogens such as fluorine, chlorine or bromine. Furthermore, R¹-R² may be substituted by amino NR⁵₂, for example dimethylamino, N-pyrrolidinyl, picolinyl, by alkoxy or aryloxy OR⁵, e.g. methoxy, ethoxy or isopropoxy, or by SiR⁶₃, e.g. trimethylsilyl, triethylsilyl, butyldimethylsilyl, tributylsilyl, tri-tert-butylsilyl, triallylsilyl, triphenylsilyl or dimethylphenylsilyl. Possible substituents R⁵ are the same carboorganic radicals as described in more detail above for R¹-R², where, if appropriate, two radicals R⁵ may be joined to form a 5- or 6-membered ring and/or be substituted by halogen. Possible radicals R⁶ for organosilicon substituents SiR⁶₃ are the same carboorganic radicals as described in more detail above for R¹-R², where, if appropriate, two radicals R⁶ may also be joined to form a 5- or 6-membered ring.

Preferred radicals R¹-R² are hydrogen, methyl, ethyl, n-propyl, isopropyl, n-butyl, isobutyl, tert-butyl, n-pentyl, n-hexyl, n-heptyl, n-octyl, benzyl or phenyl.

The substituents R¹⁷-R¹⁹, too, can be varied within wide ranges. Possible carboorganic substituents R¹⁷-R¹⁹ are, for example, the following: C₁-C₂₂-alkyl which may be linear or branched, e.g. methyl, ethyl, n-propyl, isopropyl, n-butyl, isobutyl, tert-butyl, n-pentyl, n-hexyl, n-heptyl, n-octyl, n-nonyl, n-decyl or n-dodecyl, 5- to 7-membered cycloalkyl which may in turn bear a C₁-C₁₀-alkyl group and/or C₆-C₁₀-aryl group as substituents, e.g. cyclopropyl, cyclobutyl, cyclopentyl, cyclohexyl, cycloheptyl, cyclooctyl, cyclononyl or cyclododecyl, C₂-C₂₂-alkenyl, which may be linear, cyclic or branched and in which the double bond may be internal or terminal, e.g. vinyl, 1-allyl, 2-allyl, 3-allyl, butenyl, pentenyl, hexenyl, cyclopentenyl, cyclohexenyl, cyclooctenyl or cyclooctadienyl, C₆-C₂₂-aryl which may bear further alkyl groups as substituents, e.g. phenyl, naphthyl, biphenyl, anthranyl, o-, m-, p-methylphenyl, 2,3-, 2,4-, 2,5- or 2,6-dimethylphenyl, 2,3,4-, 2,3,5-, 2,3,6-, 2,4,5-, 2,4,6- or 3,4,5-trimethylphenyl, or arylalkyl, which may bear further alkyl groups as substituents, e.g. benzyl, o-, m-, p-methylbenzyl, 1- or 2-ethylphenyl, here, if appropriate, two R¹⁷ to R¹⁹ may also be joined to form a 5-, 6- or 7-membered ring and/or two of the radicals R¹⁷-R¹⁹ may be joined to form a five-, six- or seven-membered heterocycle containing at least one atom from the group consisting of N, P, O and S and/or the organic radicals R¹⁷-R¹⁹ may also be substituted by halogens such as fluorine, chlorine or bromine. Furthermore, R¹⁷-R¹⁹ can be amino NR²⁰₂, for example dimethylamino, n-pyrrolidinyl or picolinyl, alkoxy or aryloxy OR²⁰, such as methoxy, ethoxy or isopropoxy or halogens such as fluorine, chlorine or bromine. Possible radicals R²¹ for organosilicon substituents SiR²¹₃ are the same carboorganic radicals mentioned in more detail above for R¹⁷-R¹⁹, where, if appropriate, two R²¹ may also be joined to form a 5- or 6-membered ring, e.g. trimethylsilyl, triethylsilyl, butyldimethylsilyl, tributylsilyl, tri-tert-butylsilyl, triallylsilyl, triphenylsilyl or dimethylphenylsilyl.

Preferred radicals R¹⁷-R¹⁹ are hydrogen, methyl, trifluoromethyl, ethyl, n-propyl, isopropyl, n-butyl, isobutyl, tert-butyl, n-pentyl, n-hexyl, n-heptyl, n-octyl, benzyl, phenyl, fluorine, chlorine and bromine.

Possible carboorganic substituents R²²-R³¹ are, for example, the following: C₁-C₂₂-alkyl which may be linear or branched, e.g. methyl, ethyl, n-propyl, isopropyl, n-butyl, isobutyl, tert-butyl, n=pentyl, n-hexyl, n-heptyl, n-octyl, n-nonyl, n-decyl or n-dodecyl, 5- to 7-membered cycloalkyl which may in turn bear a C₁-C₁₀-alkyl group and/or C₆-C₁₀-aryl group as substituent, e.g. cyclopropyl, cyclobutyl, cyclopentyl, cyclohexyl, cycloheptyl, cyclooctyl, cyclononyl or cyclododecyl, C₂-C₂₂-alkenyl, which may be linear, cyclic or branched and in which the double bond may be internal or terminal, e.g. vinyl, 1-allyl, 2-allyl, 3-allyl, butenyl, pentenyl, hexenyl, cyclopentenyl, cyclohexenyl, cyclooctenyl or cyclooctadienyl, C₆-C₂₂-aryl which may bear further alkyl groups as substituents, e.g. phenyl, naphthyl, biphenyl, anthranyl, o-, m-, p-methylphenyl, 2,3-, 2,4-, 2,5- or 2,6-dimethylphenyl, 2,3,4-, 2,3,5-, 2,3,6-, 2,4,5-, 2,4,6- or 3,4,5-trimethylphenyl, or arylalkyl, which may bear further alkyl groups as substituents, e.g. benzyl, o-, m-, p-methylbenzyl, 1- or 2-ethylphenyl, here, if appropriate, two R²² to R³¹ may also be joined to form a 5-, 6- or 7-membered ring and/or two of the radicals R²²-R³¹ may be joined to form a five-, six- or seven-membered heterocycle containing at least one atom from the group consisting of N, P, O and S and/or the organic radicals R²²-R³¹ may also be substituted by halogens such as fluorine, chlorine or bromine. Furthermore, R²²-R³¹ can be amino NR³²₂, for example dimethylamino, n-pyrrolidinyl or picolinyl, alkoxy or aryloxy OR³², such as methoxy, ethoxy or isopropoxy or halogens such as fluorine, chlorine or bromine. Possible radicals R³³ for organosilicon substituents SiR³³3 are the same carboorganic radicals mentioned in more detail above for R²²-R³¹, where, if appropriate, two R³³ may also be joined to form a 5- or 6-membered ring, e.g. trimethylsilyl, triethylsilyl, butyldimethylsilyl, tributylsilyl, tri-tert-butylsilyl, triallylsilyl, triphenylsilyl or dimethylphenylsilyl. Preference is given to at least one radical R²²-R³¹ being halogen such as fluorine, chlorine, bromine or iodine.

Preferred radicals R²²-R³¹ are hydrogen, methyl, trifluoromethyl, ethyl, n-propyl, isopropyl, n-butyl, isobutyl, tert-butyl, n-pentyl, n-hexyl, n-heptyl, n-octyl, benzyl, phenyl, fluorine, chlorine, bromine and iodine. In particular, R²² to R²⁵, R²⁷ and R³⁰ are each methyl, trifluoromethyl, ethyl, n-propyl, isopropyl, n-butyl, isobutyl, tert-butyl, n-pentyl, n-hexyl, n-heptyl, n-octyl, fluorine, chlorine or bromine and R²⁶, R²⁸, R²⁹ and R³¹ are each hydrogen. R²² and R²⁴ are particularly preferably each halogen such as fluorine, chlorine or bromine.

In particular, R²² and R²⁴ are identical, R²³ and R²⁵ are identical, R²⁶ and R²⁹ are identical, R²⁷ and R³⁰ are identical and R²⁸ and R³¹ are identical. This is also preferred in the preferred embodiments described further above.

Particularly preferred diimine compounds of the formula IV are 2,6-diacetylpyridinebis(2,6-dimethylphenylimine), 2,6-diacetylpyridinebis(2,4,6-trimethylphenylimine), 2,6-diacetylpyridinebis(2-chloro-6-methylphenyl), 2,6-diacetylpyridinebis(2,6-diisopropylphenylimine), 2,6-diacetylpyridinebis(2,6-dichlorophenylimine), 2,6-pyridinedicarboxaldehydebis(2,6-diisopropyl-phenylimine), diacetylpyridinebis(2,6-dichlorophenylimine), diacetylpyridinebis(2,6-difluorphenylimine), diacetylpyridinebis(2,6-dibromophenylimine).

Primary amines used here are 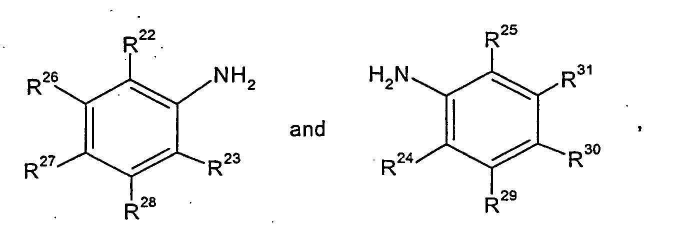 which are preferably identical. The meanings of the variables and their preferred embodiments are the same as those described above for the diimine compound IV.

The molar ratio of the dicarbonyl compound and the primary amine used is generally from 1:10 to 1:1.8, preferably from 1:5 to 1:2 and particularly preferably from 1:3 to 1:2. The order of addition of individual components is not critical. Thus, for example, the dicarbonyl compound can be initially charged and the primary amine can be added thereto.

The phosphorus pentoxide can be used as a pure substance or else as a mixture with an inert solid such as aluminum oxide, silica gel or aluminosilicate. Such mixtures are commercially available under the tradename Sicapent. The molar ratio of dicarbonyl compound to phosphorus pentoxide is preferably in the range from 1:0.1 to 1:100, preferably from 1:1 to 1:10 and particularly preferably from 1:1.5 to 1:3. The ratio of Sicapent (about 50% by weight water uptake capacity) to dicarbonyl compound is preferably in the-range from 1 g of Sicapent per 0.1 mmol of dicarbonyl compound to 1 g of Sicapent per 100 mmol of dicarbonyl compound, more preferably from 1 g of Sicapent per 1 mmol of dicarbonyl compound to 1 g of Sicapent per 10 mmol of dicarbonyl compound and particularly preferably from 1 g of Sicapent per 1.8 mmol of dicarbonyl compound to 1 g of Sicapent per 6 mmol of dicarbonyl compound.

The order of addition of the phosphorus pentoxide is not critical, but it is preferably added to the mixture of dicarbonyl compound and primary amine. The total amount of phosphorus pentoxide used can be added at the beginning of the reaction, or it can be added in a number of portions during the ongoing reaction. It has been found that the process proceeds particularly quickly when the total amount of phosphorus pentoxide is added at the beginning of the reaction.

As solvents, use is usually made of anhydrous aprotic solvents such as ethers or hydrocarbons. Suitable solvents include ethers such as diethyl ether, dibutyl ether, tetrahydrofuran or ethylene glycol ethers and aromatic and aliphatic hydrocarbons such as benzene, toluene, ethylbenzene, n-pentane, n-hexane, m-hexane, isohexane, n-heptane, n-octane, and also mixtures thereof. Preference is given to using ethers and in particular tetrahydrofuran.

It has been found to be advantageous, particularly in the preparation of diimine compounds having halogen-containing substituents on the primary amine, to carry out the synthesis under a protective gas atmosphere such as nitrogen or argon.

The reaction is generally carried out at from 18 to 150°C, preferably from 30 to 110°C and particularly preferably from 50 to 90°C. The reaction time is usually in the range from 30 minutes to 15 days, preferably from 5 hours to 5 days, particularly preferably from 8 hours to 3 days.

The work-up is carried out in a customary fashion, e.g. by removal of the solvent under reduced pressure. The product obtained can subsequently be purified by conventional methods, for example by means of chromatography of recrystallization.

The diimine compound obtained in this way can be used for the synthesis of transition metal complexes, for example iron complexes.

The process can also be used in modified form for the synthesis of diimine compounds from carbonyl-imino compounds. Since the 2^{nd} step in particular in the formation of the diimino compounds often gives low yields, the reaction of carbonyl-imino compound with primary imines in the presence of phosphorus pentoxide to form diimines is a further process according to the invention. The definition of the primary amines and the reaction conditions are the same as those given above. The molar ratio of the carbonyl-imino compound to the primary amine used is generally from 1:5 to 1:0.9, preferably from 1:2 to 1:1 and particularly preferably from 1:1.2 to 1:1. The molar ratio of carbonyl-imino compound to phosphorus pentoxide is preferably in the range from 1:0.1 to 1:50, preferably from 1:0.5 to 1:5 and particularly preferably from 1:1 to 1:2. The ratio of Sicapent (about 50% by weight water uptake capacity) to carbonyl-imino compound is preferably in the range from 1 g of Sicapent per 0.1 mmol of carbonyl-imino compound to 1 g of Sicapent per 50 mmol of carbonyl-imino compound, more preferably from 1 g of Sicapent per 0.5 mmol of carbonyl-imino compound to 1 g of Sicapent per 5 mmol of carbonyl-imino compound and particularly preferably from 1 g of Sicapent per 1 mmol of carbonyl-imino compound to 1 g of Sicapent per 3 mmol of carbonyl-imino compound.

In the process of the invention, a carbonyl-imino compound is any compound which contains at least one keto group, -C=O which is not part of a heteroaromatic ring, with aldehyde groups also being included here, and which contains at least one imine group -C=N- which is not part of a heteroaromatic ring.

Preference is given to using carbonyl-imino compounds of the formula VI 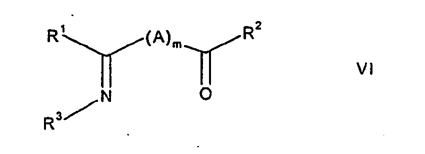 where the variables have the following meanings:
- R¹-R³: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, NR⁵₂, or a five-, six- or seven-membered heterocycle containing at least one atom from the group consisting of N, P, O and S, where the organic radicals R¹-R⁴ may also be substituted by halogens, NR⁵₂, OR⁵ or SiR⁶₃ and/or two radicals R¹-R⁴ may also be joined with one another or with A to form a ring,
- the radicals R⁵: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR⁶₃, where the organic radicals R⁵ may also be substituted by halogens and two radicals R⁵ may also be joined to form a five- or six-membered ring, and
- the radicals R⁶: are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R⁶ may also be joined to form a five- or six-membered ring,
- m: is 0 or 1,
- A: is 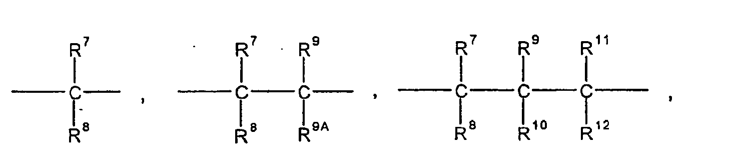 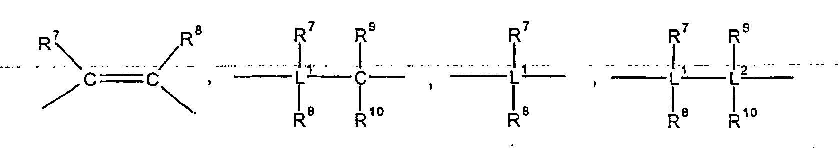 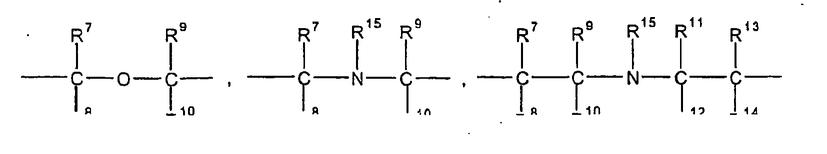 or a heteroaromatic ring system, where
- L¹-L²: are each, independently of one another, silicon or germanium,
- R⁷-R¹⁵: are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR¹⁶_{3'} where the organic radicals R⁷-R¹⁵ may also be substituted by halogens and two radicals R⁷-R¹⁵ may also be joined to form a five- or six-membered ring, and
- the radicals R¹⁶: are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R¹⁶ may also be joined to form a five- or six-membered ring.

In this process, preference is likewise given to preparing diimine compounds of the formula I using primary amines R⁴NH_{2.} The preferred embodiments of the variables are the same as those described above. This latter process is particularly useful for preparing diimine compounds having different radicals R³ and R⁴. The carbonyl-imino compound can be prepared by means of the customary synthetic methods, for example reaction of the dicarbonyl compound with p-toluenesulfonic acid in toluene.

The process of the invention allows, in particular, even primary amines having electron-withdrawing substituents to be converted into the corresponding diimine compounds in high yields. Furthermore, the time in which the conversion to the diimine proceeds to completion is significantly reduced.

A further advantage is that the process is also very suitable for producing commercial amounts. Amounts of 2 -200 kg of the diimine compound can be prepared without problems. In contrast, when molecular sieves are used, these are often attacked during stirring and then lead to problems in the work-up.

The following experimental examples serve to illustrate the invention without implying any restriction of the scope of the invention.

### Examples

### Example 1

### Preparation of 2,6-diacetylpyridinebis(2,4-dichloro-6-methylphenylanil)

45 g of 2,6-diacetylpyridine (0.276 mol), 106.8 g of 2,4-dichloro-6-methylaniline (0.607 mol) and 28 g of Sicapent were heated under reflux in 1200 ml of tetrahydrofuran for 7.5 hours and stirred under argon for a further 12 hours at room temperature. A further 28 g of Sicapent were added and the mixture was refluxed for another 10.5 hours and stirred for a further 13 hours at room temperature. The GC/MS indicates a yield of 86% of the product. The insoluble solid is filtered off and washed with tetrahydrofuran. The solvent was distilled off from the filtrate obtained in this way, the residue was admixed with 500 ml of methanol and subsequently stirred at 55°C for one hour. The suspension formed in this way was filtered and the solid obtained was washed with methanol and freed of the solvent. The filtrate was freed of the solvent once again and taken up in 200 ml of methanol and admixed with a seed crystal. The product obtained in this way was filtered off and washed with methanol. This procedure was repeated on the filtrate obtained in this way. The combined product was taken up in 800 ml of methanol, stirred for one hour, filtered and the solid was washed with ether. This gave 101.8 g of 2,6-diacetylpyridinebis(2,4-dichloro-6-methylphenylanil) in a yield of 77%.

### Example 2

### Preparation of 2,6-diacetylpyridinebis(2,4-dichloro-6-methylphenylanil)

22.5 g of 2.6-diacetylpyridine (0.138 mol), 53.39 g of 2,4-dichloro-6-methylaniline (0.303 mol) and 64 g of Sicapent were refluxed in 500 ml of tetrahydrofuran under argon for 18 hours and the mixture was subsequently cooled to room temperature. The GC/MS indicates a yield of 91.5% of the product. The work-up was carried out as described in Example 1. This gave 2,6-diacetyl-pyridinebis(2,4-dichloro-6-methylphenylanil) in a yield of 81%.

### Example 3

### Preparation of 2,6-diacetylpyridinebis(2,4-dichloro-6-methylphenylanil)

4 g of 2,6-diacetylpyridine (0.025 mol), 9.49 g of 2,4-dichloro-6-methylaniline (0.054 mol) and 8.5 g of phosphorus pentoxide were refluxed in 90 ml of tetrahydrofuran for 26 hours and stirred under argon for a further 12 hours at room temperature. The GC/MS indicates a yield of 87.3% of the product. The work-up was carried out as described in Example 1. This gave 2,6-diacetylpyridinebis(2,4-dichloro-6-methylphenylanil) in a yield of 65%.

### Comparative Example

Preparation of 2,6-diacetylpyridinebis(2,4-dichloro-6-methylphenylanil) by the method of Qian et al., Organometallics 2003, 22, 4312-4321.

65.6 g of 2,6-diacetylpyridine (0.4 mol), 170 g of 2,4-dichloro-6-methylaniline (0.483 mol), 32 g of Al-Si silica gel grade 135 and 160 g of molecular sieves (4A) were stirred in 1500 ml of toluene at 80°C for 5 hours and a further 32 g of silica gel grade 135 and 160 g of molecular sieves (4Å) were subsequently added. The mixture was stirred at 80°C for another 8 hours (the GC/MS indicates a yield of 64% of the product), the insoluble solid was filtered off and washed twice with toluene. The solvent was distilled off from the filtrate obtained in this way, the residue was admixed with 200 ml of methanol and subsequently stirred at 55°C for 1 hour. The suspension formed in this way was filtered and the solid obtained was washed with methanol and freed of the solvent. This gave 95 g of 2,6-diacetylpyridinebis(2,4-dichloro-6-methylphenylanil) in a yield of 47%.

## Claims

1. A process for preparing diimine compounds, which comprises reacting a dicarbonyl compound with primary amines in the presence of phosphorus pentoxide.

2. A process according to claim 1, wherein dicarbonyl compounds of the formula II, 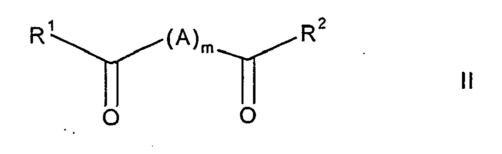 where the variables have the following meanings:
R¹-R² are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, NR⁵₂, or a five-, six- or seven-membered hetero-cycle containing at least one atom from the group consisting of N, P, O and S, where the organic radicals R¹-R² may also be substituted by halogens, NR⁵₂, OR⁵ or SiR⁶₃ and/or two radicals R¹-R² may also be joined with one another or with A to form a ring,
the radicals R⁵ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR⁶₃, where the organic radicals R⁵ may also be substituted by halogens and two radicals R⁵ may also be joined to form a five- or six-membered ring, and
the radicals R⁶ are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R⁶ may also be joined to form a five- or six-membered ring,
m is 0 or 1,
A is 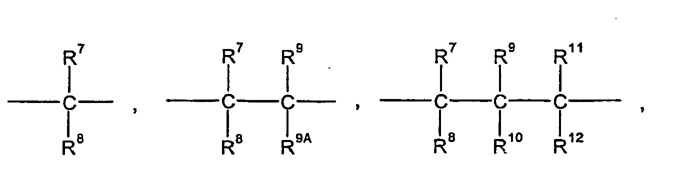 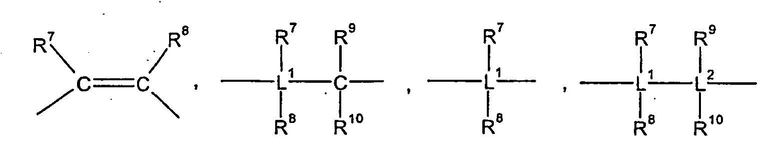 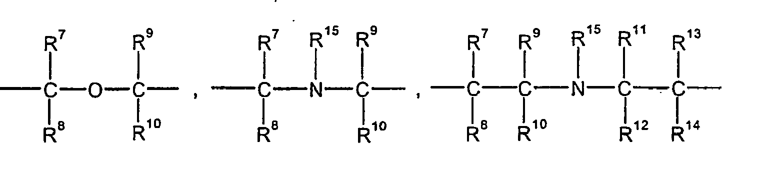 or a heteroaromatic ring system, where
L¹-L² are each, independently of one another, silicon or germanium,
R⁷-R¹⁵ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR¹⁶₃, where the organic radicals R⁷-R¹⁵ may also be substituted by halogens and two radicals R⁷-R¹⁵ may also be joined to form a five- or six-membered ring, and
The radicals R¹⁶ are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R¹⁶ may also be joined to form a five- or six-membered ring,
are used.

3. A process according to claim 2, wherein diimine compounds of the formula 1, 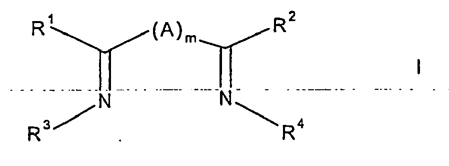 where the variables have the following meanings:
R¹-R⁴ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, NR⁵₂, or a five-, six- or seven-membered hetero-cycle containing at least one atom from the group consisting of N, P, O and S, where the organic radicals R¹-R⁴ may also be substituted by halogens, NR⁵₂, OR⁵ or SiR⁶₃ and/or two radicals R¹-R⁴ may also be joined to one another or to A to form a ring,
the radicals R⁵ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR⁶₃, where the organic radicals R⁵ may also be substituted by halogens and two radicals R⁵ may also be joined to form a five- or six-membered ring, and
the radicals R⁶ are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R⁶ may also be joined to form a five- or six-membered ring,
m is 0 or 1,
A is 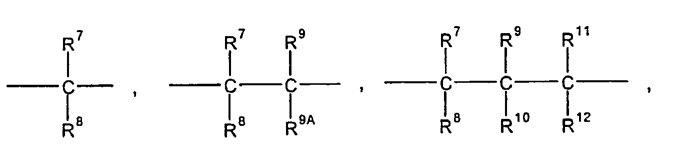 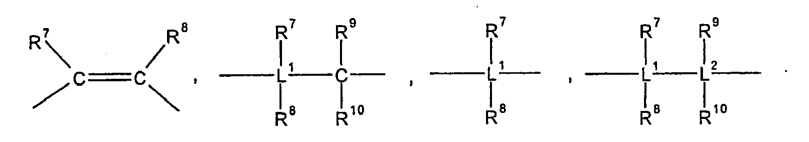 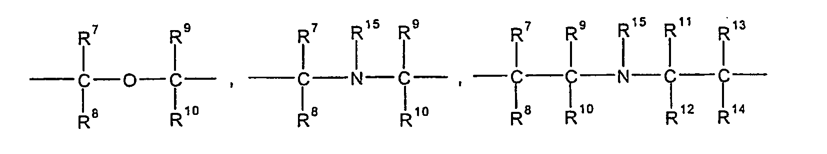 or a heteroaromatic ring system, where
L¹-L² are each, independently of one another, silicon or germanium,
R⁷-R¹⁵ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR¹⁶₃, where the organic radicals R⁷-R¹⁵ may also be substituted by halogens and two radicals R⁷-R¹⁵ may also be joined to form a five- or six-membered ring, and
the radicals R¹⁶ are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R¹⁶ may also be joined to form a five- or six-membered ring,
are prepared.

4. A process according to claim 2 or 3, wherein m is 1 and A has the formula III 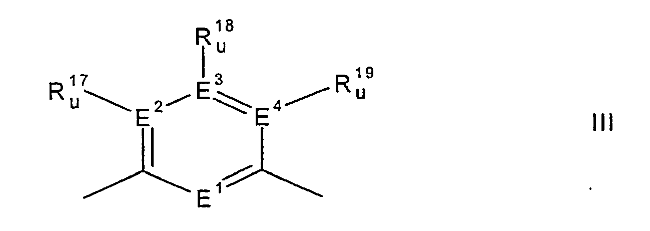 where the variables have the following meanings:
E¹ is nitrogen or phosphorus, in particular nitrogen,
E²-E⁴ are each, independently of one another, carbon, nitrogen or phosphorus, in particular carbon,
R¹⁷-R¹⁹ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, halogen, NR²⁰₂, OR²⁰, SiR²¹_{3'} where the organic radicals R¹⁷-R¹⁹ may also be substituted by halogens and/or two vicinal radicals R¹⁷-R¹⁹ may also be joined to form a five-, six- or seven-membered ring, and/or two radicals R¹⁷-R¹⁹ are joined to form a five-, six-or seven-membered hetero-cycle containing at least one atom from the group consisting of N, P, O and S,
the radicals R²⁰ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR ²¹₃, where the organic radicals R²⁰ may also be substituted by halogens and two radicals R²⁰ may also be joined to form a five- or six-membered ring, and
the radicals R²¹ are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R²¹ may also be joined to form a five- or six-membered ring,
u is 0 when E²-E⁴ is nitrogen or phosphorus and is 1 when E²-E⁴ is carbon.

5. A process according to any of claims 2 to 4, wherein m is 0.

6. A process according to claim 1, wherein diimines of the formula IV 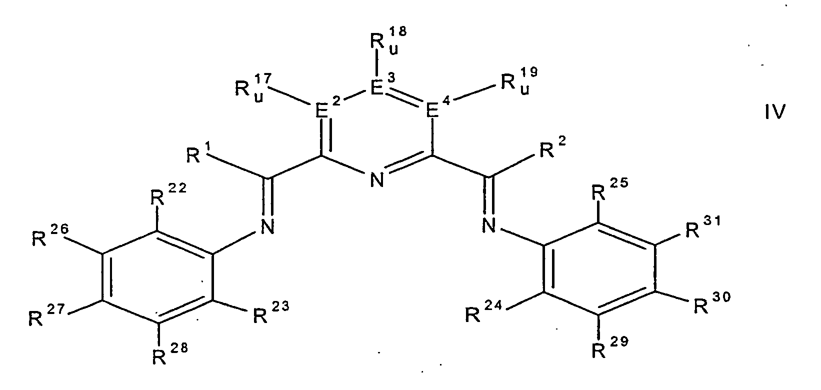 are prepared by reacting a dicarbonyl compound of the formula V 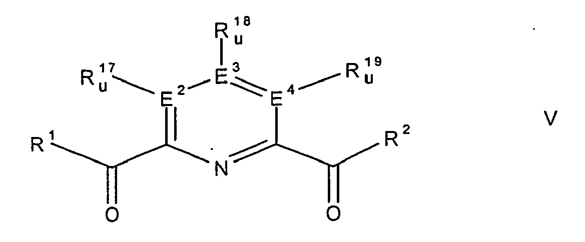 with primary amines in the presence of phosphorus pentoxide,
where the variables have the following meanings:
E²-E⁴ are each, independently of one another, carbon or nitrogen, in particular carbon,
R¹-R² are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, where the organic radicals R¹-R² may also be substituted by halogens, NR⁵₂, OR⁵ or SiR⁶₃ and/or the radicals R¹-R² may also be joined to R¹⁷-R¹⁹ to form a ring,
R¹⁷-R¹⁹ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, halogen, NR²⁰₂, OR²⁰, SiR²¹₃, where the organic radicals R¹⁷-R¹⁹ may also be substituted by halogens and/or two vicinal radicals R¹⁷-R¹⁹ may also be joined to form a five-, six- or seven-membered ring, and/or two radicals R¹⁷-R¹⁹ are joined to form a five-, six- or seven-membered hetero-cycle containing at least one atom from the group consisting of N, P, O and S,
the radicals R²⁰ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part or SiR²¹₃, where the organic radicals R²⁰ may also be substituted by halogens and two radicals R²⁰ may also be joined to form a five- or six-membered ring, and
the radicals R²¹ are each, independently of one another, hydrogen, C₁-C₂₂-alkyl, C₂-C₂₂-alkenyl, C₆-C₂₂-aryl or alkylaryl having from 1 to 10 carbon atoms in the alkyl part and 6-20 carbon atoms in the aryl part and two radicals R²¹ may also be joined to form a five- or six-membered ring,
u is 0 when E²-E⁴ is nitrogen or phosphorus and is 1 when E²-E⁴ is carbon,
R²²-R³¹ are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, halogen, NR³²₂, OR³², SiR³³₃, where the organic radicals R²²-R³¹ may also be substituted by halogens and/or two vicinal radicals R²²-R³¹ may also be joined to form a five-, six- or seven-membered ring, and/or two vicinal radicals R²²-R³¹ are joined to form a five-, six- or seven-membered heterocycle containing at least one atom from the group consisting of N, P, O and S,
the radicals R³² are each, independently of one another, hydrogen, C₁-C₂₂-alkyl, C₂-C₂₂-alkenyl, C₆-C₂₂-aryl, alkylaryl having from 1 to 10 carbon atoms in the alkyl part and 6-20 carbon atoms in the aryl part, SiR³³₃, where the organic radicals R³² may also be substituted by halogens or nitrogen- and oxygen-containing groups and two radicals R³² may also be joined to form a five- or six-membered ring, and
the radicals R³³ are each, independently of one another, hydrogen, C₁-C₂₀-alkyl, C₂-C₂₀-alkenyl, C₆-C₂₀-aryl, alkylaryl having from 1 to 10 carbon atoms in the alkyl part and 6-20 carbon atoms in the aryl part, where the organic radicals R³³ may also be substituted by halogens or nitrogen- and oxygen-containing groups and two radicals R³³ may also be joined to form a five- or six-membered ring.

7. A process according to any of claims 1 to 6, wherein the process is carried out in tetrahydrofuran.

## Patentansprüche

1. Verfahren zur Herstellng von Diiminverbindungen, **dadurch gekennzeichnet, daß** eine Dicarbonylverbindung in Anwesenheit von Phosphorpentoxid mit primären Aminen umgesetzt wird.

2. Verfahren nach Anspruch 1, **dadurch gekennzeichnet, daß** Dicarbonylverbindungen der Formel II verwendet werden, 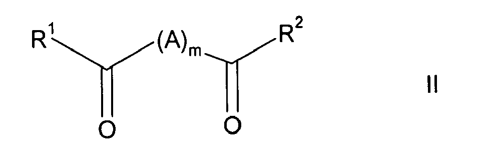 worin die Variablen folgende Bedeutung haben:
R¹-R² unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest, NR⁵₂, oder fünf-, sechs-oder siebengliedriger Heterocyclus, welcher mindestens ein Atom aus der Gruppe N, P, O oder S enthält, wobei die organischen Reste R¹-R² auch durch Halogene, NR⁵₂, OR⁵ oder SiR⁶₃ substituiert sein können und/oder zwei Reste R¹-R² miteinander oder mit A auch zu einem Ring verbunden sein können,
R⁵ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest oder SiR⁶₃, wobei die organischen Reste R⁵ auch durch Halogene substituiert sein können und zwei Reste R⁵ auch zu einem fünf- oder sechsgliedrigen Ring verbunden sein können, und
R⁶ unabhängig voneinander Wasserstoff, C₁-C₂₂-Alkyl, C₂-C₂₂-Alkenyl, C₆-C₂₂-Aryl oder Alkylaryl mit 1 bis 10 C-Atomen im Alkylrest und 6-20 C-Atomen im Arylrest und zwei Reste R⁶ auch zu einem fünf- oder sechsgliedrigen Ring verbunden sein können,
m 0 oder 1,
A 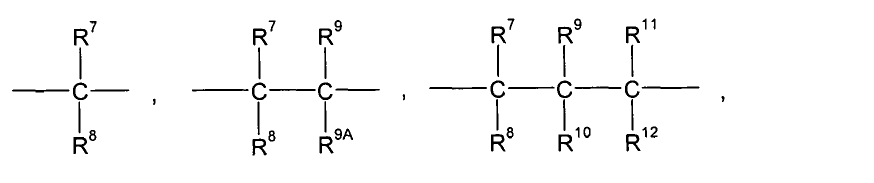 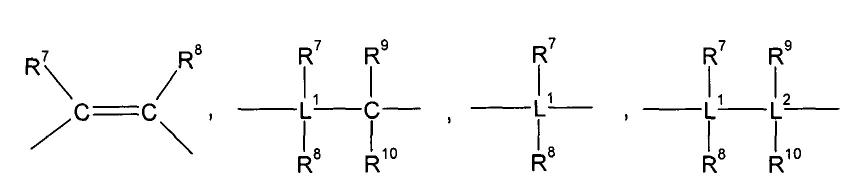 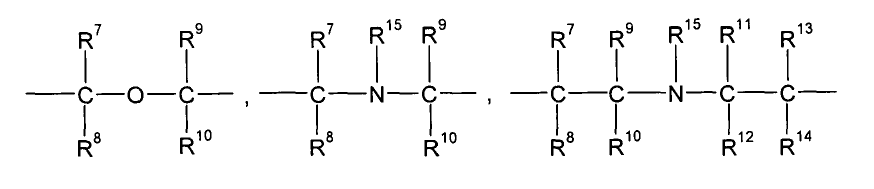 oder ein heteroaromatisches Ringsystem, wobei
L¹-L² unabhängig voneinander Silizium oder Germani-um bedeutet,
R⁷-R¹⁵ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest oder S1R¹⁶₃ bedeutet, wobei die organischen Reste R⁷-R¹⁵ auch durch Halogene substituiert sein können und zwei Reste R⁷-R¹⁵ auch zu einem fünf- oder sechsgliedrigen Ring verbunden sein können, und
R¹⁶ unabhängig voneinander Wasserstoff, C₁-C₂₂-Alkyl, C₂-C₂₂-Alkenyl, C₆-C₂₂-Aryl oder Alkylaryl mit 1 bis 10 C-Atomen im Alkylrest und 6-20 C-Atomen im Arylrest bedeutet und zwei Reste R¹⁶ auch zu einem fünf- oder sechsglied-rigen Ring verbunden sein können.

3. Verfahren nach Anspruch 2, **dadurch gekennzeichnet, daß** Diiminverbindungen der Formel I dargestellt werden, 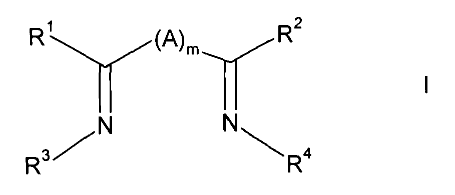 worin die Variablen folgende Bedeutung haben:
R¹-R⁴ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest, NR⁵₂, oder fünf-, sechs-oder siebengliedriger Heterocyclus, welcher mindestens ein Atom aus der Gruppe N, P, O oder S enthält, wobei die organischen Reste R¹-R⁴ auch durch Halogene, NR⁵₂, OR⁵ oder SiR⁶₃ substituiert sein können und/oder zwei Reste R¹-R⁴ miteinander oder mit A auch zu einem Ring verbunden sein können,
R⁵ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest oder SiR⁶₃, wobei die organischen Reste R⁵ auch durch Halogene substituiert sein können und zwei Reste R⁵ auch zu einem fünf- oder sechsgliedrigen Ring verbunden sein können, und
R⁶ unabhängig voneinander Wasserstoff, C₁-C₂₂-Alkyl, C₂-C₂₂-Alkenyl, C₆-C₂₂-Aryl oder Alkylaryl mit 1 bis 10 C-Atomen im Alkylrest und 6-20 C-Atomen im Arylrest und zwei Reste R⁶ auch zu einem fünf- oder sechsgliedrigen Ring verbunden sein können,
m 0 oder 1,
A 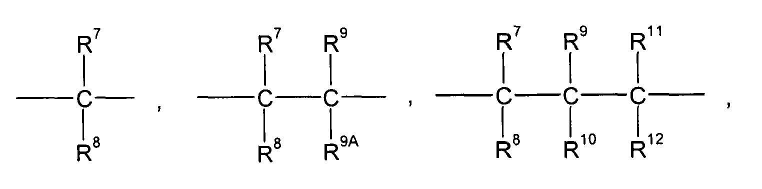 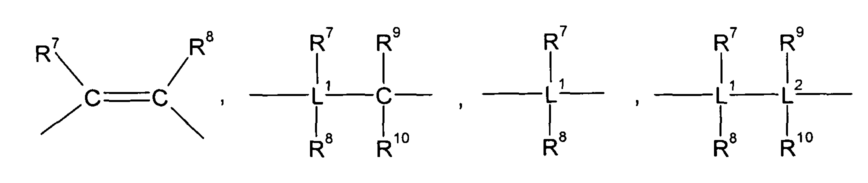 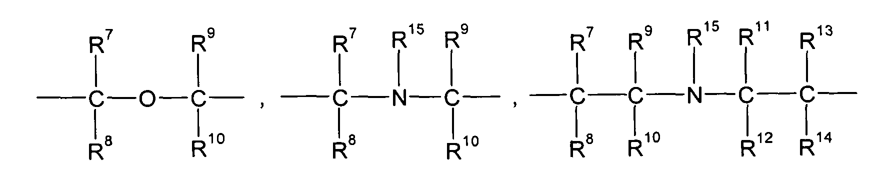 oder ein heteroaromatisches Ringsystem, wobei
L¹-L² unahängig voneinander Silizium oder Germanium bedeutet,
R⁷-R¹⁵ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest oder S1R¹⁶₃ bedeutet, wobei die organischen Reste R⁷_R¹⁵ auch durch Halogene substituiert sein können und zwei Reste R⁷-R¹⁵ auch zu einem fünf- oder sechs-gliedrigen Ring verbunden sein können, und
R¹⁶ unabhängig voneinander Wasserstoff, C₁-C₂₂-Alkyl, C₂-C₂₂-Alkenyl, C₆-C₂₂-Aryl oder Alkylaryl mit 1 bis 10 C-Atomen im Alkylrest und 6-20 C-Atomen im Arylrest bedeutet und zwei Reste R¹⁶ auch zu einem fünf- oder sechsgliedrigen Ring verbunden sein können.

4. Verfahren nach Anspruch 2 oder 3, **dadurch gekennzeichnet, daß** m gleich 1 ist und A die Formel III hat 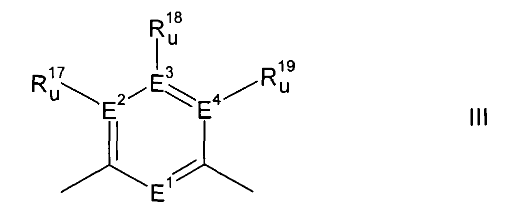 worin die Variablen folgende Bedeutung haben:
E¹ Stickstoff oder Phosphor, insbesondere Stickstoff,
E²-E⁴ unabhängig voneinander Kohlenstoff, Stickstoff oder Phosphor, insbesondere Kohlenstoff,
R¹⁷-R¹⁹ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest, Halogen, NR^{O}₂, OR²⁰, SiR²¹₃, wobei die organischen Reste R¹⁷-R¹⁹ auch durch Halogene substituiert sein können und/oder zwei vicinale Reste R¹⁷ -R¹⁹ auch zu einem fünf-, sechs- oder siebengliedrigen Ring verbunden sein können und/oder zwei Reste R¹⁷-R¹⁹ zu einem fünf-, sechs- oder sieben-gliedrigen Heterocyclus verbunden sind, welcher mindestens ein Atom aus der Gruppe N, P, O oder S enthält,
R²⁰ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest oder SiR²¹₃, wobei die organischen Reste R²⁰ auch durch Halogene substituiert sein können und zwei Reste R²⁰ auch zu einem fünf- oder sechsgliedrigen Ring verbunden sein können, und
R²¹ unabhängig voneinander Wasserstoff, C₁-C₂₂-Alkyl, C₂-C₂₂-Alkenyl, C₆-C₂₂-Aryl oder Alkylaryl mit 1 bis 10 C-Atomen im Alkylrest und 6-20 C-Atomen im Arylrest und zwei Reste R²¹ auch zu einem fünf- oder sechsgliedrigen Ring verbunden sein können,
u 0 für E²-E⁴ gleich Stickstoff oder Phosphor und 1 für E²-E⁴ gleich Kohlenstoff ist.

5. Verfahren nach den Ansprüchen 2 bis 4, **dadurch gekennzeichnet, daß** m gleich 0 ist.

6. Verfahren nach Anspruch 1, **dadurch gekennzeichnet, daß** Diimine der Formel IV 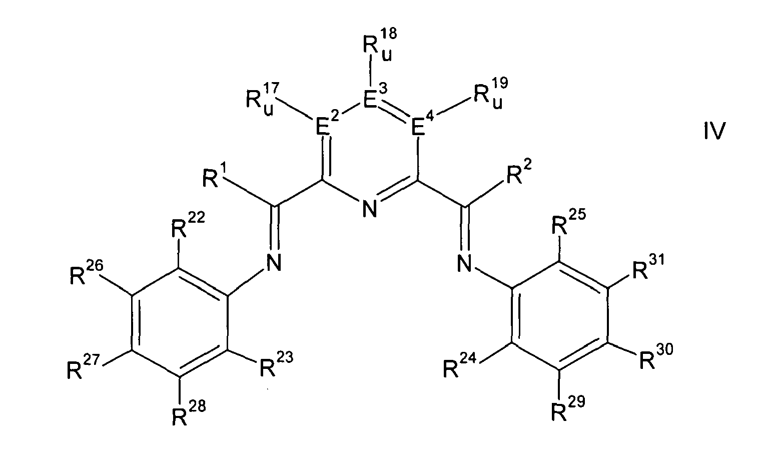 durch Umsetzung einer Dicarbonylverbindung der Formel V 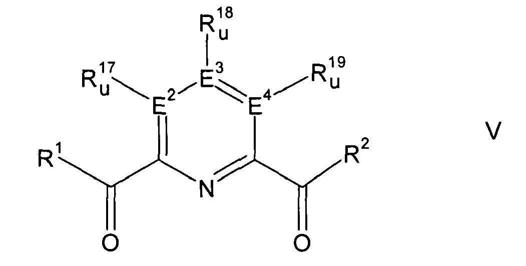 mit primären Aminen in Anwesenheit von Phosphorpentoxid hergestellt werden, wobei die Variablen folgende Bedeutung besitzen:
E²-E⁴ unabhängig voneinander Kohlenstoff oder Stickstoff, insbesondere Kohlenstoff,
R¹-R² unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest, wobei die organischen Reste R¹-R² auch durch Halogene, NR⁵₂, OR⁵ oder SiR⁶₃ substituiert sein können und/oder die Reste R¹-R² mit R¹⁷-R¹⁹ auch zu einem Ring verbunden sein können,
R¹⁷-R¹⁹ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest, Halogen, NR²⁰₂, OR²⁰, SiR²¹₃, wobei die organischen Reste R¹⁷-R¹⁹ auch durch Halogene substituiert sein können und/oder zwei vicinale Reste R¹⁷-R¹⁹ auch zu einem fünf-, sechs- oder siebengliedrigen Ring verbunden sein können und/oder zwei Reste R¹⁷-R¹⁹ zu einem fünf-, sechs- oder sieben-gliedrigen Heterocyclus verbunden sind, welcher mindestens ein Atom aus der Gruppe N, P, O oder S enthält,
R²⁰ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest oder SiR²¹₃, wobei die organischen Reste R²⁰ auch durch Halogene substituiert sein können und zwei Reste R²⁰auch zu einem fünf- oder sechsgliedrigen Ring verbunden sein können, und
R²¹ unabhängig voneinander Wasserstoff, C₁-C₂₂-Alkyl, C₂-C₂₂-Alkenyl, C₆-C₂₂-Aryl oder Alkylaryl mit 1 bis 10 C-Atomen im Alkylrest und 6-20 C-Atomen im Arylrest und zwei Reste R²¹ auch zu einem fünf- oder sechsgliedrigen Ring verbunden sein können,
u 0 für E²-E⁴ gleich Stickstoff oder Phosphor und 1 für E²-E⁴ gleich Kohlenstoff ist,
R²²-R³¹ unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest, Halogen, NR ³² ₂, OR³², SiR³³₃, wobei die organischen Reste R²²-R³¹ auch durch Halogene substituiert sein können und/oder zwei vicinale Reste R²² -R ³¹ auch zu einem fünf-, sechs- oder siebengliedrigen Ring verbunden sein können und/oder zwei vicinale Reste R²²-R³¹ zu einem fünf-, sechs- oder siebengliedrigen Heterocyclus verbunden sind, welcher mindestens ein Atom aus der Gruppe N, P, O oder S enthält,
R³² unabhängig voneinander Wasserstoff, C₁-C₂₂-Alkyl, C₂-C₂₂-Alkenyl, C₆-C₂₂-Aryl, Alkylaryl mit 1 bis 10 C-Atomen im Alkylrest und 6-20 C-Atomen im Arylrest, SiR³³₃, wobei die organischen Reste R³² auch durch Halogene oder stickstoff- und sauerstoffhaltige Gruppen substituiert sein können und zwei Reste R³² auch zu einem fünf- oder sechsgliedrigen Ring verbunden sein können, und
R³³ unabhängig voneinander Wasserstoff, C₁-C₂₀-Alkyl, C₂-C₂₀-Alkenyl, C₆-C₂₀-Aryl, Alkylaryl mit 1 bis 10 C-Atomen im Alkylrest und 6-20 C-Atomen im Arylrest, wobei die organischen Reste R³³ auch durch Halogene oder stickstoff-und sauerstoffhaltige Gruppen substituiert sein können und zwei Reste R³³ auch zu einem fünf- oder sechsgliedrigen Ring verbunden sein können.

7. Verfahren nach den Ansprüchen 1 bis 6, **dadurch gekennzeichnet, daß** das Verfahren in Tetrahydrofuran durchgeführt wird.

## Revendications

1. Procédé pour la préparation de composés de diimine, qui comprend la réaction d'un composé dicarbonylé avec des amines primaires en présence de pentoxyde de phosphore.

2. Procédé selon la revendication 1, dans lequel on utilise des composés dicarbonylés de formule II, 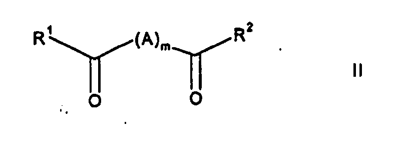 où les variables ont les significations suivantes:
R¹-R² sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle, NR⁵₂, ou un hétérocycle à cinq, six ou sept chaînons comprenant au moins un atome choisi dans le groupe consistant en N, P, O et S, tandis que les radicaux organiques R¹-R² peuvent également être substitués par des halogènes, NR⁵₂, OR⁵ ou SiR⁶₃ et/ou deux radicaux R¹-R² peuvent également être reliés entre eux ou avec A pour former un cycle,
les radicaux R⁵ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle ou SiR⁶₃, tandis que les radicaux organiques R⁵ peuvent également être substitués par des halogènes et deux radicaux R⁵ peuvent également être reliés pour former un cycle à cinq ou six chaînons, et
les radicaux R⁶ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle et deux radicaux R⁶ peuvent également être reliés pour former un cycle à cinq ou six chaînons,
m est 0 ou 1,
A est 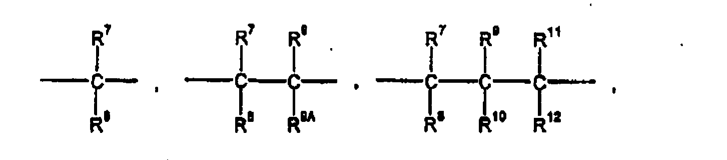 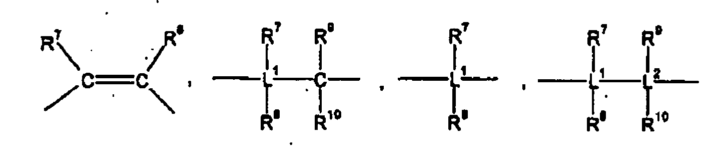 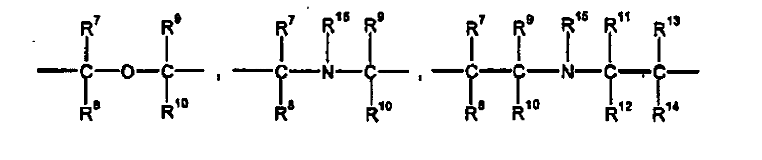 ou un système cycle hétéroaromatique, tandis que
L¹-L² sont chacun, indépendamment l'un de l'autre, silicium ou germanium,
R⁷-R¹⁵ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle ou SiR¹⁶₃, tandis que les radicaux organiques R⁷-R¹⁵ peuvent également être substitués par des halogènes et deux radicaux R⁷-R¹⁵ peuvent également être reliés pour former un cycle à cinq ou six chaînons, et
les radicaux R¹⁶ sont chacun, indépendamment l'un de l'autre, hydrogène, alkyle en C₁-C₂₂, alcényle en C₂-C₂₂, aryle en C₆-C₂₂ ou alkylaryle ayant de 1 à 10 atomes de carbone dans la partie alkyle et 6-20 atomes de carbone dans la partie aryle et deux radicaux R¹⁶ peuvent également être reliés pour former un cycle à cinq ou six chaînons.

3. Procédé selon la revendication 2, dans lequel on prépare des composés de diimine de formule I, 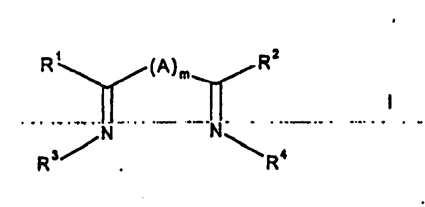 où les variables ont les significations suivantes:
R¹-R⁴ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle, NR⁵₂, ou un hétérocycle à cinq, six ou sept chaînons comprenant au moins un atome choisi dans le groupe consistant en N, P, O et S, tandis que les radicaux organiques R¹-R⁴ peuvent également être substitués par des halogènes, NR⁵₂, OR⁵ ou SiR⁶₃ et/ou deux radicaux R¹-R⁴ peuvent également être reliés entre eux ou avec A pour former un cycle,
les radicaux R⁵ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle ou SiR⁶₃, tandis que les radicaux organiques R⁵ peuvent également être substitués par des halogènes et deux radicaux R⁵ peuvent également être reliés pour former un cycle à cinq ou six chaînons, et
les radicaux R⁶ sont chacun, indépendamment l'un de l'autre, hydrogène, alkyle en C₁-C₂₂, alcényle en C₂-C₂₂, aryle en C₆-C₂₂ ou alkylaryle ayant de 1 à 10 atomes de carbone dans la partie alkyle et 6-20 atomes de carbone dans la partie aryle et deux radicaux R⁶ peuvent également être reliés pour former un cycle à cinq ou six chaînons,
m est 0 ou 1,
A est 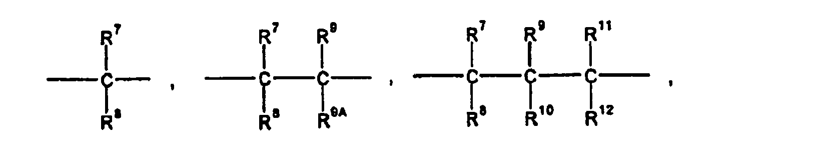 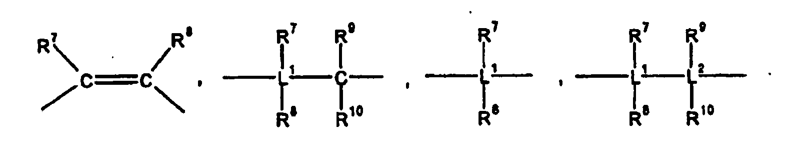 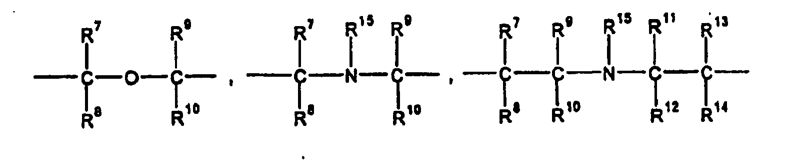 ou un système cycle hétéroaromatique, tandis que
L¹-L² sont chacun, indépendamment l'un de l'autre, silicium ou germanium,
R⁷-R¹⁵ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle ou SiR¹⁶₃, tandis que les radicaux organiques R⁷-R¹⁵ peuvent également être substitués par des halogènes et deux radicaux R⁷-R¹⁵ peuvent également être reliés pour former un cycle à cinq ou six chaînons, et
les radicaux R¹⁶ sont chacun, indépendamment l'un de l'autre, hydrogène, alkyle en C₁-C₂₂, alcényle en C₂-C₂₂, aryle en C₆-C₂₂ ou alkylaryle ayant de 1 à 10 atomes de carbone dans la partie alkyle et 6-20 atomes de carbone dans la partie aryle et deux radicaux R¹⁶ peuvent également être reliés pour former un cycle à cinq ou six chaînons.

4. Procédé selon la revendication 2 ou 3, dans lequel m est 1 et A à la formule III 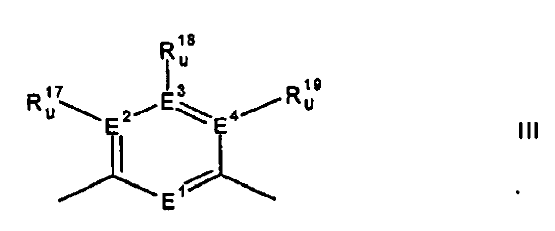 où les variables ont les significations suivantes:
E¹ est l'azote ou le phosphore, en particulier l'azote,
E²-E⁴ sont chacun, indépendamment l'un de l'autre, carbone, azote ou phosphore, en particulier carbone,
R¹⁷-R¹⁹ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle, halogène, NR²⁰₂, OR²⁰, SiR²¹₃, tandis que les radicaux organiques R¹⁷-R¹⁹ peuvent également être substitués par des halogènes et/ou deux radicaux vicinaux R¹⁷-R¹⁹ peuvent également être reliés pour former un cycle à cinq, six ou sept chaînons, et/ou deux radicaux R¹⁷-R¹⁹ sont reliés pour former un hétérocycle à cinq, six ou sept chaînons, contenant au moins un atome choisi dans le groupe consistant en N, P, O et S,
les radicaux R²⁰ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle ou SiR²¹₃, tandis que les radicaux organiques R²⁰ peuvent également être substitués par des halogènes et deux radicaux R²⁰ peuvent également être reliés pour former un cycle à cinq ou six chaînons, et
les radicaux R²¹ sont chacun, indépendamment l'un de l'autre, hydrogène, alkyle en C₁-C₂₂, alcényle en C₂-C₂₂, aryle en C₆-C₂₂ ou alkylaryle ayant de 1 à 10 atomes de carbone dans la partie alkyle et 6-20 atomes de carbone dans la partie aryle et deux radicaux R²¹ peuvent également être reliés pour former un cycle à cinq ou six chaînons,
u est 0 lorsque E²-E⁴ est l'azote ou le phosphore et est 1 lorsque E²-E⁴ est le carbone.

5. Procédé selon l'une quelconque des revendications 2 à 4, dans lequel m est 0.

6. Procédé selon la revendication 1, dans lequel on prépare des diimines de formule IV 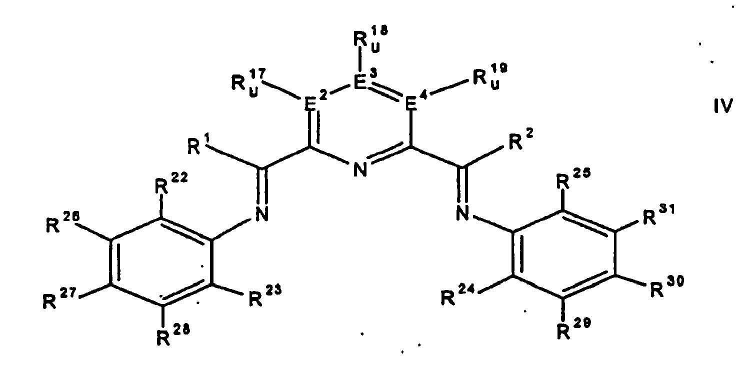 en faisant réagir un composé dicarbonylé de formule V 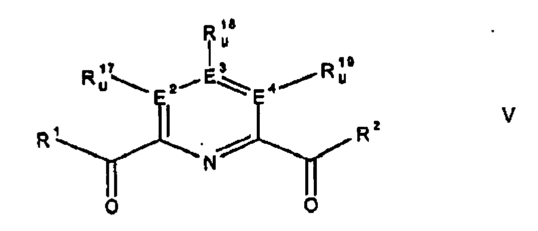 avec des amines primaires en présence de pentoxyde de phosphore, tandis que les variables ont les significations suivantes:
E²-E⁴ sont chacun, indépendamment l'un de l'autre, carbone ou hydrogène, en particulier carbone,
R¹-R² sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle, tandis que les radicaux organiques R¹-R² peuvent également être substitués par des halogènes NR⁵₂, OR⁵ ou SiR⁶₃ et/ou les radicaux R¹-R² peuvent également être reliés à R¹⁷-R¹⁹ pour former un cycle,
R¹⁷-R¹⁹ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle, halogène, NR²⁰₂, OR²⁰, SiR²¹₃, tandis que les radicaux organiques R¹⁷-R¹⁹ peuvent également être substitués par des halogènes et/ou deux radicaux vicinaux R¹⁷-R¹⁹ peuvent également être reliés pour former un cycle à cinq, six ou sept chaînons, et/ ou deux radicaux R¹⁷-R¹⁹ sont reliés pour former un hétérocycle à cinq, six ou sept chaînons, contenant au moins un atome choisi dans le groupe consistant en N, P, O et S,
les radicaux R²⁰ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle ou SiR²¹₃, tandis que les radicaux organiques R²⁰ peuvent également être substitués par des halogènes et deux radicaux R²⁰ peuvent également être reliés pour former un cycle à cinq ou six chaînons, et
les radicaux R²¹ sont chacun, indépendamment l'un de l'autre, hydrogène, alkyle en C₁-C₂₂, alcényle en C₂-C₂₂, aryle en C₆-C₂₂ ou alkylaryle ayant de 1 à 10 atomes de carbone dans la partie alkyle et 6-20 atomes de carbone dans la partie aryle et deux radicaux R²¹ peuvent également être reliés pour former un cycle à cinq ou six chaînons,
u est 0 lorsque E²-E⁴ est l'azote ou le phosphore et est 1 lorsque E²-E⁴ est le carbone.
R²²-R³¹ sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle, halogène, NR³²₂, OR³², SiR³³₃, tandis que les radicaux organiques R²²-R³¹ peuvent également être substitués par des halogènes et/ou deux radicaux vicinaux R²²-R³¹ peuvent également être reliés pour former un cycle à cinq, six ou sept chaînons, et/ou deux radicaux vicinaux R²²-R³¹ sont reliés pour former un hétérocycle à cinq, six ou sept chaînons, contenant au moins un atome choisi dans le groupe consistant en N, P, O et S,
les radicaux R³² sont chacun, indépendamment l'un de l'autre, hydrogène, alkyle en C₁-C₂₂, alcényle en C₂-C₂₂, aryle en C₆-C₂₂, alkylaryle ayant de 1 à 10 atomes de carbone dans la partie alkyle et 6-20 atomes de carbone dans la partie aryle, SiR³³₃, tandis que les radicaux organiques R³² peuvent également être substitués par des halogènes et des groupes contenant de l'azote et de l'oxygène et deux radicaux R³² peuvent également être reliés pour former un cycle à cinq ou six chaînons, et
les radicaux R³³ sont chacun, indépendamment l'un de l'autre, hydrogène, alkyle en C₁-C₂₀, alcényle en C₂-C₂₀, aryle en C₆-C₂₀, alkylaryle ayant de 1 à 10 atomes de carbone dans la partie alkyle et 6-20 atomes de carbone dans la partie aryle, tandis que les radicaux organiques R³³ peuvent également être substitués par des halogènes ou des groupes contenant de l'azote et de l'oxygène et deux radicaux R³³ peuvent également être reliés pour former un cycle à cinq ou six chaînons.

7. Procédé selon l'une quelconque des revendications 1 à 6, dans lequel le procédé est mis en oeuvre dans du tétrahydrofuranne.
